# EUROPEAN PATENT SPECIFICATION

(11) **EP 1 699 769 B1**
(45) Date of publication and mention of the grant of the patent: **04.04.2007**
(21) Application number: 04809258.9
(22) Date of filing: 23.12.2004
(51) Int. Cl.: C07D 277/72

(54) **METHOD OF OBTAINING 2-MERCAPTOBENZOTHIAZOLE**
VERFAHREN ZUR GEWINNUNG VON 2-MERCAPTOBENZOTHIAZOL
PROCEDE SERVANT A PREPARER 2-MECAPTOBENZOTHIAZOL

(30) Priority: 23.12.2003 SK 16162003
(43) Date of publication of application: 13.09.2006
(73) Proprietor: DUSLO, a.s., SK-92703 Sala (SK)
(72) Inventor: KRIZANOVIC, Karol, 900 21 Sväty Jur (SK); MUNTAGOVA, Lubica, 851 01 Bratislava (SK)
(74) Representative: Bachrata, Magdaléna
(86) International application number: PCT/SK2004/000018
(87) International publication number: WO 2005/061471

(56) References cited:
- EP-A- 0 475 226
- US-A- 4 371 698
- US-A- 4 647 669

## Description

### Technical Field

The invention concerns a method of obtaining 2-mercaptobenzothiazole from a melt of the raw product, wherein the melt contains 2-mercaptobenzothiazole, unreacted raw materials, intermediate products and pitches.

### Background Art

2-Mercaptobenzothiazole (hereinafter 2-MBT) is a basic member of the group of benzothiazole accelerators of sulphur vulcanisation of unsaturated rubbers and, simultaneously, it is the decisive basic raw material for industrial production of further important benzothiazole accelerators, like *N-*cyclohexylbenzothiazolesulphene amide, *N-tert*-butylbenzothiazolesulphene amide, *N,N*-dicyclohexylbenzothiazolesulphene amide, *N*-oxydiethylenebenzothiazolesulphene amide and others.

On an industrial scale, 2-mercaptobenzothiazole is prepared by highpressure synthesis at high temperature directly from aniline, carbon disulphide and sulphur. In this way a product is obtained, the quality of which does not meet qualitative requirements for application, and in most cases neither for its subsequent processing to sulphene amide accelerators. Therefore, the raw 2-mercaptobenzothiazole must be refined.

At present, following techniques are used for industrial purification of 2-mercaptobenzothiazole:
- pre-precipitation using alkali and mineral acids; the disadvantages are a high content of salts (in the process, there arises at least one mole of inorganic salts per one mole of 2-mercaptobenzothiazole), problems with separation and subsequent disposal of water-insoluble pitchy substances, and also the necessity of distilling off several defined substances present in the raw melt (aniline, benzothiazole, phenylisothiocyanate) using water steam;
- crystallization of the raw melt in a suitable solvent, like some chlorinated alkanes and alkenes, toluene, nitrobenzene and others. Major disadvantage of the above technique is a high content of 2-mercaptobenzothiazole in mother liquors which arise in the course of crystallization and from which the product is recovered using a relatively demanding process, if the loss in yield is to be lowered. No less important disadvantage of the process is the use of an auxiliary starting substance with all disadvantages associated therewith.

A combination of the above mentioned processes, where 2-mercaptobenzothiazole is extracted by a diluted aqueous alkali solution from the raw melt dissolved in a suitable solvent and subsequently precipitated by a mineral acid, may moderately improve technical-ecological parameters of the product purification process, but it does not solve the above given disadvantages of the involved processes in principle.

The disadvantage of refining the raw product by means of a further solvent is eliminated by using a suitable raw material for 2-mercaptobenzothiazole production. Impurities are removed in such a way that the reaction product is extracted by carbon disulphide or by an emulsion of carbon disulphide in water. This process is described in US 2,090,233 and 3,030,373. US 3,031,073 also describes in detail purification of the melt by an emulsion water/carbon disulphide with addition of a surfactant, but also crystallization of the raw product with 1.5-fold amount of carbon disulphide in an autoclave at 140 °C under pressure during 45 minutes. Moreover, it uses not only carbon disulphide for crystallization, but after thickening the mother liquors from the refinement it tries to utilize the pitchy substances contained therein in further synthesis in such a way that after supplementing the rest of raw materials - aniline and sulphur - the mixture is charged into a pressure reactor. The process is repeated twice on the whole, while quality of the product decreases with further recycling of the pitches. If at least a part of pitches is not withdrawn from the process, their concentration in the system increases, thus causing lowering of qualitative parameters of the product, in consequence of which this is not suitable for the production of sulphene amide accelerators.

The latest solution, which utilizes carbon disulphide refinement of 2-mercaptobenzothiazole, where the filtrate is simultaneously recycled in the process, is described in DE 4,028,473 (US 5,367,082). A 2-mercaptobenzothiazole melt, obtained in a batch reactor in a conventional manner, is partially cooled by adding carbon disulphide and under the pressure of the resulting hydrogen sulphide it is cooled down to 25 °C. After depressurising the product is filtered and washed with carbon disulphide. Carbon disulphide mother liquors and wash fractions are thickened under vacuum and charged together with the corresponding amount of aniline and sulphur into the reactor. With successive repeating of the above method the yield increases to an average value of 98.7 %, while the content of the active substance and the melting temperature gradually decrease. The authors do not state directly in the examples any removing of a part of mother liquors from the system, but in the invention description they mention that the number of cycles is essentially unlimited, if in each cycle a small part of the mother liquor is removed.

Despite indisputable advantages of the refinement by means of carbon disulphide, also this process has some essential shortcomings:
- necessity to use pressure equipment for crystallization,
- necessity to handle in the process large amounts (even 10-fold with respect to 2-mercaptobenzothiazole) of easily inflammable and toxic carbon disulphide,
- increased energy intensity at distilling off carbon disulphide from the filtrates after crystallization, i.e. at thickening the mother liquors to 10 to 15 % of the original volume.

From FR 2,450,828 (CS 231 971), FR 2,565,977 (US 4,647,669), there is known refinement of 2-mercaptobenzothiazole in aniline. There is stated a possibility of recycling the used recycling medium after the refinement under the condition that one must remove from the system as many impurities as many impurities enter the system together with the reaction product.

The basis of the method described in FR 2,450,828 (CS 231 971) consists in that the raw product melt containing 83.25 % of 2-mercaptobenzothiazole, 2.75 % of aniline, 5 % of benzothiazole and 9 % of pitches is dissolved in 2.5-fold amount of the used ("thickened") aniline (containing 50.7 % of aniline + 1.24 % of benzothiazole + 18.8 % of 2-mercaptobenzothiazole + 29.25 % of side substances from the previous purifying operations. After cooling down the product crystallizes, is filtered and several times washed with nearly triple amount of aniline. After removing the wash aniline from the cake containing 2-mercaptobenzothiazole, a product with the content of active substance of 98.9 % and a yield of up to 97.6 %, with respect to the starting 2-mercaptobenzothiazole in the raw product melt. Aniline filtrates are thickened to the starting volume and used in a further purifying operation, the process being repeated with a further batch of melt. To keep an equilibrium between impurities and product, approximately 9 % of mother liquors from primary filtration are withdrawn from the system before mixing with wash aniline and thickening the used aniline to the required volume. From the separated residue, aniline with benzothiazole are distilled off and returned to crystallization; the non-distilling residue (containing pitches and 2-mercaptobenzothiazole) may be removed from the system and partially or completely recycled into the reactor.

The above described refinement method has several disadvantages which make its successful application in practice more difficult or even impossible:
- thickening of crystallization filtrates and wash aniline to the starting volume, i.e. thickening to 1/3, which is energy-intensive and subjecting aniline and substances dissolved in the refining medium to permanent heat stress which may contribute to further formation of pitches,
- washing the filtration cake containing 2-mercaptobenzothiazole by pure aniline leads to dissolution of the product, i.e. to yield losses. Product dissolution creates small channels in the layer of the filtration cake, and inhomogeneous cake causes imperfect washing away of pitch residues from 2-mercaptobenzothiazole and lowering the content of active substance in the product. In these cases it is better to wash the filtration cake in a liquid medium and filter the product again.
- despite the declared removing of 9 % of the filtrate from crystallization benzothiazole will cumulate in the system, because the volatile fractions (aniline and benzothiazole) distilled off from it return back to the refining process, thus making worse the refinement effect,
- complete recycling of the distillation residue into the rector means, instead of removing it from the process, returning all removed pitches back into the system, their accumulation and making the refinement of the raw product impossible.

A method according to FR 2,565,977 (US 4,647,669) is based on the same principle, it differs just by the method of isolation of the pure product and of its drying. These are processes without recycling the used aniline filtrates into the reactor for 2-mercaptobenzothiazole production. It should be sufficient to remove calculable amount of the used filtrate to retain the refining ability of the system, but in fact, this does not work. The description includes also a remark on possible recycling of the distillation residue (obtained from the removed amount of the filtrate) through the reactor, but this possibility is not described in more detail in the examples.

The aim of the present invention is to eliminate the need to remove amine hydrochloride by filtration, by crystallization from petroleum ether, as well as to obtain 2-mercaptobenzothiazole with required quality and yield in a simpler way.

### Disclosure of Invention

The above given aim is achieved by obtaining 2-mercaptobenzothiazole from a melt of the raw product, prepared by the reaction of aniline, carbon disulphide and sulphur, through pressure synthesis, wherein the melt contains 2-mercaptobenzothiazole, unreacted raw materials, intermediate products and pitches, by a method according to the present invention.

For the purpose of the present invention, under the term "liquid phase" we shall understand "mother liquor", "aniline filtrate" or "liquid phase" after separating 2-mercaptobenzothiazole by filtration, decantation or centrifuging, always containing aniline.

A melt of the 2-mercaptobenzothiazole raw product, where the melt contains 2-mercaptobenzothiazole, unreacted raw materials, intermediate products and pitches, is prepared by the reaction of aniline, carbon disulphide and sulphur through pressure synthesis. The subject matter of the invention consists in that the 2-mercaptobenzothiazole raw product from the pressure reactor is in the first batch dissolved in an excess (in an approximately double amount) of pure aniline. By cooling down the solution, crystals of 2-mercaptobenzothiazole are precipitated, which are separated and the liquid phase (filtrate from crystallization; F_{K}) is used in the next batch. The obtained cake of crystallized (pre-purified) product is processed with a minimum amount of fresh aniline and pure product is separated from the resulting mixture. The obtained liquid phase F_{R} from final purification is recycled by charging it into the next crystallization.

In the next batch, approximately 1/3 of the liquid phase from crystallization is charged into the reactor for 2-mercaptobenzothiazole preparation and is supplemented, with respect to the content of aniline, by sulphur and carbon disulphide. The obtained melt of the 2-mercaptobenzothiazole raw product is dissolved in the remainder (approximately 2/3) of the liquid phase from crystallization of the preceding batch and is supplemented by the whole amount of liquid phase F_{R} from final purification, also from the preceding batch, and possibly also by aniline.

After crystallization the cake is processed by mixing (dispersing) in pure or regenerated aniline or in a suitable liquid phase. Aniline or suitable liquid phase enter the process in counterflow - they first finally purify the crystallized product in the wash separating operation, then the liquid phase from wash separation enters the raw product crystallization and finally, the liquid phase from the 2-mercaptobenzothiazole raw product crystallization is charged into the reactor for 2-mercaptobenzothiazole synthesis.

After several recycles the composition of the system (reaction medium) becomes steady.

An advantage of the method according to the present invention consists in that recycling of a greater amount of aniline mother liquors from the refinement directly into the reactor (instead of pure aniline) with simultaneous removing of defined amounts of these liquors from the system does not influence negatively the quality of the 2-mercaptobenzothiazole raw product obtained from the reactor, neither the quality of the product obtained by its refinement by aniline or the corresponding liquid phase, and this quality is retained also in the course of multiple repeats at a level of 98 % of active substance, even without necessity to thicken the aniline mother liquors.

A melt of raw 2-mercaptobenzothiazole obtained directly from the reactor contains besides benzothiazole also further defined chemical compounds, intermediate reaction products, for example thiocarbanilide, anilidobenzothiazole, as well as unreacted raw materials - aniline and sulphur. The actual content of pitches (undefined substances) is lower than 9 %. All stated substances contained in the mother liquors from the refinement may, if returned into the reactor, convert to the product or they maintain chemical equilibrium in favour of the product, suppress side reactions and increase the yield of the process. It is advantageous for the process to recycle them into the reactor in the greatest possible extent.

Working conditions in a batch or tube reactor (temperature, pressure, reaction time) used for preparation of the raw 2-mercaptobenzothiazole from aniline filtrates do not differ from standard conditions of 2-mercaptobenzothiazole synthesis from pure raw materials.

It has been found that it is advantageous if at the refinement (final purification) of the raw product the wash separation or washing the filtration cake of 2-mercaptobenzothiazole after crystallization by aniline is replaced by wash separation or washing by a solution of aniline with pure 2-mercaptobenzothiazole. This solution does not dissolve the product and does not cause formation of small channels in the filtration cake - the filtration cake is homogeneous and impurities are washed out over the whole cross-section of the cake. In this way pure product of comparable quality is obtained by a simpler method.

### Examples of invention embodiments

The following examples illustrate in more detail, but do not limit the nature of the invention. The examples describe the course of the process after stabilization of the reaction medium in equilibrium (which is characterized by the stable composition of the streams), which is achieved by several preceding recycles.

### Example 1

### (without recycling the aniline filtrates)

93.13 g of aniline, 31.42 g of sulphur and 80.67 g of carbon disulphide were charged into a pressure 300 ml reactor. Under conditions usual for this synthesis (220 to 300 °C/6 to 11.1 MPa) a melt of 2-mercaptobenzothiazole raw product was prepared. After reaction the reactor was cooled down to 180 to 200 °C and the reactor content was purged at 200 °C by nitrogen stream, removing the volatile fractions.

155 g of purged melt were obtained (containing 92.3 % of 2-mercaptobenzothiazole, 1.72 % of benzothiazole, 2.14 % of sulphur and 3.84 % of pitches) and 8.9 g of volatile fractions (a mixture of aniline and benzothiazole).

The melt was hot dissolved in 250 g of aniline, 2-mercaptobenzothiazole was crystallized by cooling, and after filtration and drying 114.6 g of the product (i.e. 68.5 % of theoretical yield with respect to aniline charged into the reactor, and 80.1 % yield with respect to the 2-mercaptobenzothiazole content in the raw melt) were obtained, containing 98.4 % of active substance.

### Example 2

### (After stationary state was reached by multiple recycling of a part of aniline filtrates)

100 g of aniline filtrate from crystallization (containing 78 % of aniline, 9.5 % of 2-mercaptobenzothiazole) were charged into a pressure 300 ml reactor together with 27 g of sulphur, 67 g of carbon disulphide and 9 g of volatile fractions from the preceding purging of the melt (containing 30 % of aniline and 65 % of benzothiazole). Under conditions usual for this synthesis (220 to 300 °C/6 to 11.1 MPa) a melt of the raw product was prepared. After reaction the reactor was cooled down to 180 to 200 °C and the reactor content was purged at 200 °C by nitrogen stream, removing the volatile fractions.

162.3 g of purged melt were obtained containing 91.0 % of 2-mercaptobenzothiazole, 2.0 % of benzothiazole, 1.5 % of sulphur, 0.9 % of anilidobenzothiazole, 0.1 % of thiocarbanilide, 0.07 % of 2-methylbenzothiazole and 4.43 % of pitches, which melt was dissolved in a mixture of 222 g of liquid phase (filtrate) from the preceding crystallization and 200 g of liquid phase (filtrate) from the preceding final purification by wash separation.

After cooling down the crystallized 2-mercaptobenzothiazole product was filtered off, from ~ 295 g of the filtrate 5 % were removed out of the process (waste), 100 g were used as a charge of the next batch in the reactor and the remaining filtrate was used in the next crystallization. Wet aniline cake containing 2-mercaptobenzothiazole was wash separated in 91 g of pure aniline, filtered off, aniline was removed from it by wash separation in hot water and by purging with water steam, and dried. The filtrate from wash separation, consisting in general only of aniline and 2-mercaptobenzothiazole, was used in the next crystallization.

136.9 g of the product containing 98.2 % of active substance, i.e. 91 % yield with respect to 2-mercaptobenzothiazole in the melt, were obtained.

### Example 3 - comparative

The weights were identical with those in Example 1, the amount of obtained purged melt and crystallization conditions were the same as in Example 1, only the wet cake was not wash separated in pure aniline, but was washed with 91 g of aniline directly on the filter and dried. The wash filtrate was returned into the process.

139.9 g of the product were obtained containing 96.3 % of active substance, which represent 91.2 % yield with respect to 2-mercaptobenzothiazole in the melt.

### Example 4

20 g of aniline, 80 g of aniline filtrate from crystallization of the same composition as in Example 1 (78 % of aniline, 9.5 % of 2-mercaptobenzothiazole) were charged into the reactor together with 28 g of sulphur, 71 g of carbon disulphide and 9 g of benzothiazole from purging (volatile fractions). The reactor was heated to working temperature, after reaction it was cooled to 180 to 200 °C, accumulated hydrogen sulphide was released through a pressure control valve and the reactor content was evacuated at 200 °C. At a reduced pressure of 20 Torr and a temperature of 200 °C, volatile fractions were distilled off from the melt.

161 g of purged melt were obtained containing 92.2 % of 2-mercaptobenzothiazole, 1.8 % of benzothiazole, 1.3 % of sulphur, 0.7 % of anilidobenzothiazole, 0.1 % of thiocarbanilide, 0.05 % of 2-methylbenzothiazole and 3.85 % of unidentified substances. The melt was dissolved in a mixture of 222 g of filtrate from the preceding crystallization and 200 g of filtrate from the preceding refinement. After cooling down the crystallized 2-mercaptobenzothiazole was filtered off, from the filtrate 20 % were removed out of the process, 80 g were separated for the next batch in the reactor and the rest for the next crystallization. Wet aniline product cake was wash separated in 115 g of pure aniline, filtered off, cleared of aniline, and dried. The filtrate from wash separation was used in the next crystallization.

129.2 g of the product with purity of 99.4 % were obtained, i.e. 86.5 % yield with respect to 2-mercaptobenzothiazole in the melt.

### Example 5

Into a tube reactor of 5 mm diameter and 15 m length (V = 295.5 ml) with a pressure control valve at the end 83 g of aniline filtrate of the composition as in Example 1 and further 22.5 g of sulphur, 56 g of carbon disulphide and 8 g of benzothiazole from purging were charged (169.5 g altogether). The retention time was 1 hour 44 minutes. Under conditions usual for this synthesis (250 to 300 °C/6 to 11.1 MPa) a melt of raw 2-mercaptobenzothiazole was obtained which was after removing hydrogen sulphide purged with nitrogen at 200 °C. After 1 hour and 12 minutes 162 g of purged melt were obtained containing 91.2 % of 2-mercaptobenzothiazole and having further composition similar to that of the raw product from Example 1.

Crystallization and final purification were performed identically as in Example 1. 136.5 g of the product with purity of 98.5 % were obtained, representing 91 % yield with respect to 2-mercaptobenzothiazole in the melt.

### Example 6

The weights of raw materials and recycled media in the reactor were identical with those in Example 1, also the amount of obtained purged melt and crystallization conditions were the same.

The wet cake was after filtration finally purified by washing with 91 g of saturated solution of pure 2-mercaptobenzothiazole in aniline directly on the filter and dried. The wash filtrate was returned into the process.

146.9 g of the product with purity of 97.8 % were obtained, i.e. 97.3 % yield of the refinement with respect to the entered 2-mercaptobenzothiazole.

## Claims

1. Method of obtaining 2-mercaptobenzothiazole from a melt of the raw product prepared by the reaction of aniline, carbon disulphide and sulphur by pressure synthesis in a reactor, where the melt contains 2-mercaptobenzothiazole, unreacted raw materials, intermediate products and pitches, **characterized in that** after reaching a stationary state of the reaction medium it includes the following steps:
a) crystallization of the 2-mercaptobenzothiazole raw product from an aniline solution,
b) dividing the liquid phase (F_{K}) from crystallization from step a) in three parts,
c) removing one part of the liquid phase (F_{K1}) from crystallization from step a) out of the process,
d) returning the second part of the liquid phase (F_{K2}) from crystallization from step a) into the reactor for preparation of the raw product and supplementing it with sulphur and carbon disulphide with respect to aniline,
e) final purification of the crystallized 2-mercaptobenzothiazole from step a) in the aniline liquid phase and separation of the pure 2-mercaptobenzothiazole,
f) using the third part of the liquid phase (F_{K3}) from crystallization from step a), supplemented with the liquid phase (F_{R}) from final purification from step e) and possibly with aniline for crystallization of a further batch of the 2-mercaptobenzothiazole raw product,
g) using the liquid phase (F_{R}) from final purification from step f), together with a part of the liquid phase (F_{K3}) from step e), possibly with aniline, for crystallization of the 2-mercaptobenzothiazole raw product,
h) repeating steps a) to g).

2. Method according to claim 1, **characterized in that** in the step c) 5 to 20 % of the liquid phase from crystallization are removed.

3. Method according to claim 1, **characterized in that** t the crystallization of the 2-mercaptobenzothiazole raw product is performed from the aniline liquid phase from the preceding crystallization and/or from the aniline liquid phase from final purification and/or from pure aniline.

4. Method according to claim 1, **characterized in that** the aniline liquid phase in step f) for final purification of 2-mercaptobenzothiazole is pure aniline or regenerated aniline or aniline liquid phase from the preceding final purification or a solution of 2-mercaptobenzothiazole in aniline or any combination thereof.

5. Method according to claim 4, **characterized in that** t the aniline liquid phase in step f) for final purification of 2-mercaptobenzothiazole is a saturated solution of 2-mercaptobenzothiazole in aniline.

6. Method according to claim 1, **characterized in that** 2-mercaptobenzothiazole obtained in step a) is finally purified by wash separation.

7. Method according to claim 1, **characterized in that** t 2-mercaptobenzothiazole obtained in step a) is finally purified by washing.

8. Method according to claim 7, **characterized in that** 2-mercaptobenzothiazole obtained in step a) is finally purified by washing on a filter.

9. Method according to claim 7, **characterized in that** t 2-mercaptobenzothiazole obtained in step a) is finally purified by washing in a centrifuge.

## Patentansprüche

1. Methode der Gewinnung von 2-Merkaptobenzothiazol aus der Schmelze vom Rohprodukt, hergestellt durch die Reaktion von Anilin, Schwefelkohlenstoff und Schwefel durch Drucksynthese in einem Reaktor, wobei die Schmelze 2-Merkaptobenzothiazol, nicht abreagierte Rohmateriale, Zwischenprodukte und Peche enthält,
**dadurch gekennzeichnet,**
**dass** sie nach der Erreichung von stationärem Zustand vom Reaktionsmedium folgende Schritte umfasst:
a) Kristallisierung vom 2-Merkaptobenzothiazolrohprodukt aus einer Anilinlösung,
b) Teilung der flüssigen Phase (F_{K}) aus der Kristallisierung vom Schritt a) in drei Teile,
c) Entfernung von einem Teil der flüssigen Phase (F_{K1}) aus der Kristallisierung vom Schritt a) aus dem Prozess,
d) Rückführung von zweitem Teil der flüssigen Phase (F_{K2}) aus der Kristallisierung vom Schritt a) in den Reaktor zur Herstellung des Rohproduktes und dessen Nachfüllung mit Schwefel und Schwefelkohlenstoff in Bezug auf Anilin,
e) Endreinigung vom kristallisierten 2-Merkaptobenzothiazol aus dem Schritt a) in einer flüssigen Anilinphase und Separation von reinem 2-Merkaptobenzothiazol,
f) Benutzung des dritten Teiles der flüssigen Phase (F_{K3}) aus der Kristallisierung vom Schritt a), ergänzt mit der flüssigen Phase (F_{R}) aus der Endreinigung vom Schritt e) und gegebenenfalls mit Anilin für Kristallisierung von einem weiteren Ansatz von 2-Merkaptobenzothiazolrohprodukt,
g) Benutzung der flüssigen Phase (F_{R}) aus der Endreinigung vom Schritt f) zusammen mit einem Teil der flüssigen Phase (F_{K3}) vom Schritt e), gegebenenfalls mit Anilin, für Kristallisierung vom 2-Merkaptobenzothiazolrohprodukt,
h) Wiederholung der Schritte a) bis g).

2. Methode nach Anspruch 1,
**dadurch gekennzeichnet,**
**dass** man im Schritt c) 5 bis 20 % der flüssigen Phase aus der Kristallisierung entfernt.

3. Methode nach Anspruch 1,
**dadurch gekennzeichnet,**
**dass** die Kristallisierung vom 2-Merkaptobenzothiazolrohprodukt aus der flüssigen Anilinphase aus der vorherigen Kristallisation und/oder aus der flüssigen Anilinphase aus der Endreinigung und/oder aus reinem Anilin erfolgt.

4. Methode nach Anspruch 1,
**dadurch gekennzeichnet,**
**dass** die flüssige Anilinphase im Schritt f) zur Endreinigung vom 2-Merkaptobenzothiazol reines Anilin oder regeneriertes Anilin oder flüssige Anilinphase aus der vorherigen Endreinigung oder eine Lösung vom 2-Merkaptobenzothiazol in Anilin oder eine Kombination davon ist.

5. Methode nach Anspruch 4,
**dadurch gekennzeichnet,**
**dass** die flüssige Anilinphase im Schritt f) zur Endreinigung vom 2-Merkaptobenzothiazol eine gesättigte Lösung des 2-Merkaptobenzothiazols in Anilin.

6. Methode nach Anspruch 1,
**dadurch gekennzeichnet,**
**dass** das im Schritt a) gewonnene 2-Merkaptobenzothiazol ist endgültig durch Aufschlämmung gereinigt.

7. Methode nach Anspruch 1,
**dadurch gekennzeichnet,**
**dass** das im Schritt a) gewonnene 2-Merkaptobenzothiazol ist endgültig durch Auswaschen gereinigt.

8. Methode nach Anspruch 7,
**dadurch gekennzeichnet,**
**dass** das im Schritt a) gewonnene 2-Merkaptobenzothiazol ist endgültig durch Auswaschen auf einem Filter gereinigt.

9. Methode nach Anspruch 7,
**dadurch gekennzeichnet,**
**dass** das im Schritt a) gewonnene 2-Merkaptobenzothiazol ist endgültig durch Auswaschen in einer Zentrifuge gereinigt.

## Revendications

1. Méthode d'extraction du 2-mercaptobenzothiazole d'une coulée du produit brut préparé par une réaction de synthèse sous pression entre l'aniline, le carbone disulfide et le souffre, réalisée dans un réacteur alors que la coulée contient 2-mercaptobenzothiazole, les substrats non réagis, les produits intermédiaires et les poix,
**caractérisée en ce qu'** après l'obtention d'état stationnaire dans le milieu réactionnel elle comprend les étapes suivantes:
a) cristallisation du produit brut de 2-mercaptobenzothiazole en solution d'aniline,
b) division de la phase aqueuse (F_{K}) obtenue après la cristallisation pendant l'étape a) en trois parties,
c) élimination d'une partie de la phase liquide (F_{K1}) obtenue après la cristallisation pendant l'étape a) du procédé,
d) retour de la deuxième partie de la phase liquide (F_{K2}) obtenue par la cristallisation pendant l'étape a) dans le réacteur pour la préparation du produit brut et son complètement par le souffre et le carbone disulfide par rapport au contenu d'aniline,
e) purification du 2-mercaptobenzothiazole cristallisé obtenu par l'étape a) dans la phase liquide d'aniline et séparation du 2-mercaptobenzothiazole pur,
f) utilisation de la troisième partie de la phase liquide (F_{K3}) provenant de la cristallisation dans l'étape a) complétée par la phase liquide (F_{R}) de la purification obtenu dans l'étape e), éventuellement par aniline, pour la cristallisation du batch suivant du produit brut de 2-mercaptobenzothiazole,
g) utilisation de la phase liquide (F_{R}) après la purification dans l'étape f) avec une partie de la phase liquide (F_{K3}) obtenue dans l'étape e) éventuellement avec l'aniline pour la cristallisation du produit brut de 2-mercaptobenzothiazole,
h) répétition des étapes a) jusqu'à g).

2. La méthode selon la revendication 1, **caractérisée en ce que** dans l'étape c) 5 jusqu'a 20 % de la phase liquide après la cristallisation sont éliminés.

3. La méthode selon la revendication 1, **caractérisée en ce que** la cristallisation du produit brut de 2-mercaptobenzothiazole est réalisée en phase liquide d'aniline de la cristallisation précédente et/ou en phase liquide d'aniline après la purification et/ou d'aniline pure.

4. La méthode selon la revendication 1, **caractérisée en ce que** comme la phase liquide d'aniline de l'étape f) pour purification du 2-mercaptobenzothiazole sont utilisés l'aniline pure, l'aniline régénérée ou la phase d'aniline de la purification précédente ou une solution du 2-mercaptobenzothiazole dans l'aniline ou leur combinaison quelconque.

5. La méthode selon la revendication 1, **caractérisée en ce que** comme la phase liquide d'aniline dans l'étape f) pour la purification du 2-mercaptobenzothiazole une solution saturée de 2-mercaptobenzothiazole dans l'aniline est utilisée.

6. La méthode selon la revendication 1, **caractérisée en ce que** le 2-mercaptobenzothiazole obtenu dans l'étape a) est purifié par une désagrégation dans un liquide.

7. La méthode selon la revendication 1, **caractérisée en ce que** le 2-mercaptobenzothiazole obtenu dans l'étape a) est purifié par un lavage.

8. La méthode selon la revendication 1, **caractérisé en ce que** le 2-mercaptobenzothiazole obtenu dans l'étape a) est purifié par un lavage sur un filtre.

9. La méthode selon la revendication 1, **caractérisé en ce que** le 2-mercaptobenzothiazole obtenu dans l'étape a) est purifié par un lavage dans une centrifugeuse.
